# EUROPEAN PATENT SPECIFICATION

(11) **EP 3 595 512 B1**
(45) Date of publication and mention of the grant of the patent: **03.01.2024**
(21) Application number: 18711278.4
(22) Date of filing: 12.03.2018
(51) Int. Cl.: A61B 5/00, A61B 5/08, A61B 5/097

(54) **SYSTEMS AND METHODS FOR ACTIVE CANCELLATION OF PRESSURE PULSES**
SYSTEME UND VERFAHREN ZUR AKTIVEN AUFHEBUNG VON DRUCKIMPULSEN
SYSTÈMES ET PROCÉDÉS D'ANNULATION ACTIVE D'IMPULSIONS DE PRESSION

(30) Priority: 17.03.2017 US 201762472963 P
(43) Date of publication of application: 22.01.2020
(73) Proprietor: Koninklijke Philips N.V., 5656 AG Eindhoven (NL)
(72) Inventor: GERETY, Eugene Peter, 5656 AE Eindhoven (NL)
(74) Representative: Philips Intellectual Property & Standards
(86) International application number: PCT/EP2018/055991
(87) International publication number: WO 2018/166928

(56) References cited:
- US-A1- 2001 031 224
- US-A1- 2002 162 397
- US-A1- 2004 062 684
- US-A1- 2008 119 753
- US-A1- 2008 119 754
- US-A1- 2008 127 977
- SHYAM SIVARAMAKRISHNAN ET AL: "Dynamic Model Inversion Techniques for Breath-by-Breath Measurement of Carbon Dioxide from Low Bandwidth Sensors", IEEE SENSORS JOURNAL, IEEE SERVICE CENTER, NEW YORK, NY, US, vol. 10, no. 10, 10 June 2010 (2010-06-10) , pages 1637-1646, XP011334806, ISSN: 1530-437X, DOI: 10.1109/JSEN.2010.2047942

## Description

### FIELD

The following relates generally to the monitoring arts, respiration arts, pressure pulsation monitoring arts, pressure pulsation cancellation arts, gaseous concentration measurement arts, and related arts.

### BACKGROUND

In sidestream respiration gas monitors (RGMs), also known as diverting RGMs, a sample of respiration gas is drawn from a patient down a sample tube to a measuring area of the RGM (respiration gas monitor) where any of a variety of techniques can be used to measure the concentration of one or more components of the respiration gas including, but not limited to, carbon dioxide (CO₂), oxygen (O₂), nitrous oxide (N₂O), and halogenated agents such as halothane, enflurane, isoflurane, sevoflurane and desflurane. Patterns of variation in the concentrations of these gas components can have clinical significance in the treatment of patients. Accordingly, it is desired to provide a consistent, accurate temporal record of the monitored gas concentration to aid in the diagnosis and treatment of a variety of conditions. To this end, the manner of gas sampling can have a great influence on the performance and accuracy of an RGM.

An example of such a respiration gas monitor can be found in US2001/031224 to Labuda. Typically, a small diaphragm pump or similar air-moving device is used to create the gas flow from the patient to the measuring area. By the reciprocating nature of their operation, such pumps tend to move the sample gas in a pulsatile manner, producing significant pressure variation, i.e. ripple, in the sample line, especially in the vicinity of the pump. Other types of mechanical air pumps similarly tend to introduce a pressure ripple due to the cyclical nature of the pumping mechanism, usually at the cycle frequency or a multiple thereof, e.g. twice the cycle frequency. These pressure variations, depending upon their magnitude and frequency, can interfere with flow rate measurement and gas concentration measurement.

Two methods that have been used previously to deal with these pressure pulsation (i.e. ripple) include: (1) providing an air "reservoir" to absorb and attenuate the pulsation, effectively reducing the amplitude of the pulsations to manageable levels; and (2) low-pass filtering the pressure-drop measurement to attenuate the pulsation. Both of these methods have disadvantages. The reservoir approach, while effective, adds significant physical volume, which is disadvantageous where space is at a premium, and there is significant desire to reduce the physical volume of RGMs.

The low-pass filtering approach adds little physical volume, but does nothing to attenuate or eliminate the pressure pulsation in the gas sampling system. Since the pulsations can be very large compared to the pressure drop associated with flow, this creates a risk of pressure sensor saturation unless a sensor with a very wide pressure sensing range is employed. If the sensor saturates, the low-pass method produces an error in flow rate measurement, but selecting a wide-range sensor (compared to the desired measurement) usually results in poor measurement accuracy unless a very expensive, high-accuracy sensor is chosen. Further, this technique permits the unattenuated pressure pulsations to appear in the measurement area, producing errors in the measurement of gas concentrations.

Improvements disclosed herein address the foregoing and other disadvantages of respiratory gas monitoring systems, methods, and the like.

### BRIEF SUMMARY

In accordance with one illustrative example, a respiration gas monitor device includes a pump connected to draw a flow of respired air, a pressure sensor is connected to measure an air pressure signal responsive to the flow of respired air, and a pressure transducer. Electrical circuitry is operatively connected to measure flow across the pressure sensor. A gas component sensor is arranged to monitor a target gas in the flow of respired air.

In accordance with another illustrative example, a device for attenuating or eliminating pressure ripple in a respiration gas monitor is provided. The device includes a pump configured to draw respired air from a measurement area, and a constrictor through which at least a portion of the respired air drawn by the pump moves. At least one pressure sensor is configured to measure a pressure value of air flowing through the constrictor. A ripple cancellation device is configured to attenuate or eliminate at least one pressure ripple in the respired air flowing through the constrictor.

In accordance with another illustrative example, a respiratory gas monitoring method includes: drawing, with a pump, respired air through a measurement area, at least a portion of the respired air moving through a constrictor; measuring, with at least one pressure sensor, a pressure signal of air flowing through the constrictor; attenuating or eliminating, with a ripple cancellation device, at least one pressure ripple in the respired air flowing through the constrictor; and measuring, with a measurement device, a target gas in the flow of expired air.

One advantage resides in removing pressure pulsations in an air pressure signal.

Another advantage resides in measuring gases in air without pressure pulsations.

Another advantage resides in controlling flow in a pump by removing pressure pulsations.

Another advantage resides in measure concentrations of different gases in respired air.

Further advantages of the present disclosure will be appreciated to those of ordinary skill in the art upon reading and understand the following detailed description. It will be appreciated that a given embodiment may provide none, one, two, or more of these advantages.

### BRIEF DESCRIPTION OF THE DRAWINGS

The present disclosure may take form in various components and arrangements of components, and in various steps and arrangements of steps. The drawings are only for purposes of illustrating the preferred embodiments. The claims define the matter for which protection is sought in terms of the technical features of the invention.
FIGURE 1 diagrammatically illustrates a respiration gas monitor device according to one aspect.
FIGURE 2 is an exemplary flow chart of the calibration process of the device of FIGURE 1.

### DETAILED DESCRIPTION

In RGMs, the respiration gas flow is typically regulated to a relatively constant rate to avoid temporal distortion of the gas concentration record (i.e., waveform). This flow regulation is often accomplished by introducing a constrictor (such as an orifice or a capillary tube) into the gas flow path and controlling the pump drive level is controlled to maintain a constant pressure drop across the constrictor. Since the pressure drop is a direct function of the flow rate, maintaining a constant pressure drop produces a constant flow rate. In the presence of pump-induced pressure pulsations, however, the amplitude of the pulsations in the pressure drop can be large, and may even exceed the magnitude of the flow-induced pressure drop, which can be problematic for accuracy of the flow rate measurement and control.

Another problem potentially introduced by pressure pulsations in the sample line is that the pressure pulsations appear on the gas sample in the measuring area of the RGM. The gas concentration measurements can be affected by the temperature and pressure of the gas in the measuring area, so any significant pressure pulsations can interfere with the accuracy of those measurements.

The following relates to an improvement in Respiration Gas Monitor (RGM) devices employing a sidestream (i.e. diverting) flow arrangement in which a pump is provided to draw sample gas from the main respiration circuit into a side stream feeding the RGM device. The pump produces a negative pressure (a "vacuum") that draws the flow into the side stream. A diaphragm pump is commonly used, which produces a constant or average negative pressure on which is superimposed a "ripple" pressure variation component. The average negative pressure can be on the order of 1 psi (= 6.89kPa in SI units) while the ripple may be comparable, e.g. 0.5 psi (= 3.45kPa in SI units). Other types of pumps also operate using a cyclical pumping cycle that similarly typically introduces a pressure ripple. This ripple introduces pressure variations in the sampling chamber. Since the CO2 or other gas measurement is pressure-dependent, the ripple can introduce respiration gas measurement errors.

The following describes improved RGM devices in which an audio transducer is provided to reduce or eliminate the pressure ripple introduced by the pump. In some illustrative embodiments, the transducer control circuit includes a differential pressure sensor to measure the pressure drop over a constrictor (i.e., orifice or capillary tube) and a high-pass or bandpass filter to filter the measured pressure to extract the ripple. The high-pass filtered signal is inverted and applied to drive the audio transducer to generate an opposing ripple that reduces or cancels the ripple produced by the pump. The transducer is thus used to provide a more uniform pressure output from the fluid pump.

There are many ways of sensing flow, including but not limited to: hot-wire, ultrasonic sensing by differential time delay, and measuring the pressure drop across an obstacle in the flow path, among others. The following describes the pressure drop/obstacle method, but other flow sensing techniques could be adapted. With the pressure drop/obstacle method of flow sensing, a "constrictor" device is used. The constrictor is essentially any obstacle placed in the flow path. The obstruction to flow produces a relative drop in pressure on the "lee" side of the obstacle when air (gas) passes by it. This difference in pressure is a function of flow. The pressure sensor measures the difference in pressure before and after the obstruction, thereby producing an electrical signal responsive to and representative of airflow past the obstacle.

The most common constrictor type is an orifice. The orifice is very inexpensive, but has the disadvantage that it is highly nonlinear and highly sensitive to temperature and various gas properties. Another type of constrictor is a capillary tube, which is highly linear and much less sensitive to temperature and other sources of error. The capillary tube, used in this way, is sometimes referred to as a "linear flow converter".

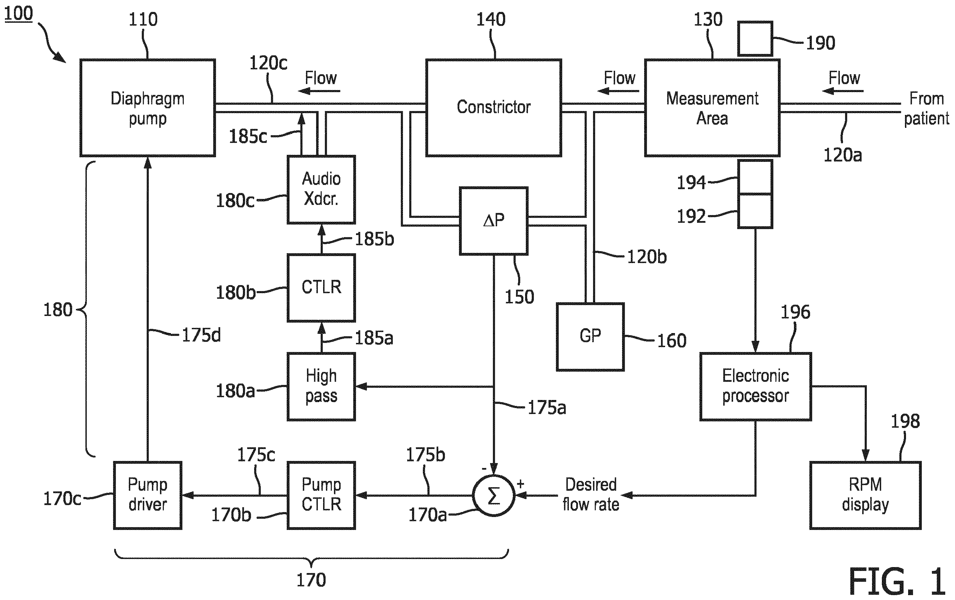

With reference now to FIGURE 1, a schematic illustration of a respiratory gas monitor (RGM) device **100** including components for attenuating or eliminating a pressure ripple. The RGM device **100** includes an air-moving device, such as a diaphragm pump **110,** connected to draw a flow of respired air by a patient. In a typical sidestream configuration, the respired air is drawn from a nasal cannula, tracheal intubation, or other patient accessory. The RGM device **100** also includes a flow constrictor **140** through which at least a portion of the respired air drawn by the pump moves. The pump **110** is connected to draw a flow of respiration gas from a patient through a sample tube segment **120a** into a measurement area **130.** The air then flows to the constrictor **140,** which is disposed between the pump **110** and the measurement area **130.** The pump **110** then receives the air from the constrictor **140.** Ultimately, the air drawn by the pump **110** may be discharged to the ambient air, optionally after passing through a scrubbing device (not shown). A sample tube segment **120b** provides connection of a differential pressure sensor **150** to measure differential pressure across the constrictor **140,** and connection of an optional gauge pressure sensor **160.** In some examples, the constrictor **140** can be a capillary tube or an orifice.

The device **100** also includes the at least one pressure sensor **150** connected to measure an air pressure signal responsive to the flow of respired air through the constrictor **140.** For example, the flow of respiration gas through the constrictor **140** creates a pressure drop across the constrictor. In this example, the pressure sensor **150** is a differential pressure sensor that measures the pressure decrease from the inlet to the outlet of the constrictor **140.** The pressure sensor **150** is configured to measure this pressure drop and produce a differential pressure signal **175a** representative of the pressure drop across the constrictor **140,** (and hence representative of the rate of gas flow through the constrictor). In some examples, the pump **110** is connected to draw the flow of respired air through the constrictor **140** and the pressure sensor **150** is connected to measure the air pressure signal indicating a pressure change across the constrictor. The constrictor **140** and the pressure sensor **150** serve to maintain a constant rate of airflow. A control mechanism, such as a pump controller (not shown) drives the pump **110** to maintain a constant pressure drop across the constrictor (as measured by the differential pressure sensor).The device **100** optionally further includes a gauge sensor **160** arranged to monitor pressure of the respired air. The gauge pressure sensor **160** is configured to measure the pressure at the outlet of the measurement area **130,** and hence represents pressure of the respired air in the measurement area **130.** This gauge pressure measurement is optionally used in the calculation of the concentrations of the target gas (e.g. carbon dioxide in the case of the RGM device **100** being a capnometer) in the respiration gas sample present in the measurement area **130** at any given time. In the illustrative example, a target gas measurement device is an optical measurement device that includes an infrared light source **190,** a light detector **192,** and a bandpass filter **194.** The infrared light source **190** is arranged to launch infrared light that is transmitted through the measurement area **130,** and more particularly through the flow of respired air through the measurement area **130.** The bandpass filter **194** is arranged to filter the infrared light to pass a wavelength absorbed by the target gas (that is, the bandpass filter **194** has a passband that encompasses an absorption line of the target gas, e.g. the 4.3 micron absorption line of carbon dioxide in the case of a capnometer). The light detector **192** is arranged to detect the infrared light after being transmitted through the flow of respired air and filtered by the bandpass filter **194.** The target gas concentration or partial pressure is computed, e.g. by a microprocessor or other electronic processor **196,** based on the detected infrared light intensity. A higher concentration of the target gas in the respired air produces more absorption and hence a reduced transmitted and bandpass-filtered infrared light intensity. Optionally, the determination of the concentration or partial pressure of target gas takes into account known factors that can affect the measurement, such as the pressure of the respired air as measured by the gauge pressure sensor **160,** and/or a calibration infrared intensity measured in the absence of the respired air flow. The electronic processor **196** may also optionally compute a clinically significant value, such as end-tidal carbon dioxide (etCO₂) in the case of the RGM device **100** implementing capnography. The target gas measurement and/or derived clinical value such as etCO₂ is displayed on an RGM display **198** (e.g. an LCD display showing the target gas concentration or partial pressure and/or the derived clinical quantity as a real-time numeric value, and/or as a trend line, or so forth). Additionally or alternatively, the data may be ported off the RGM device **100** via a wired or wireless communication link (not shown, e.g. a wired or wireless Ethernet link, a Bluetooth link, et cetera). The electronic processor **196** may also optionally perform various RGM device control functions, such as outputting the desired flow rate to the flow control mechanism **170.**

The illustrative optical target gas measurement device **190, 192, 194** is merely an illustrative example, and more generally any type of target gas measurement device may be employed to measure the concentration or partial pressure of the target gas in the respired air flowing through the measurement area **130.**

In some examples, the pump **110** is a reciprocating or cyclically operating device that moves air (i.e., respiration gas) in a pulsatile fashion, thereby producing significant pressure pulsations (i.e. pressure ripple) in the tubing segment **120c.** If these pressure pulsations are transmitted via the constrictor **140** and sample tube segment **120b** to the measurement area **130,** then they can lead to measurement error. The amplitude of the pulsations is likely to be reduced somewhat after passing through the tubing segments **120c, 120b** and the constrictor **140,** but this attenuation may not be enough to prevent a significant pulsation waveform to appear in measurements made by the gauge pressure sensor **160.** These pulsations can create errors in the measured concentrations of the components of the respiration gas sample present in the measurement area **130.**

The device **100** can also include electronic circuitry configured to control various operations thereof (e.g., flow control, pulse cancellation, and the like). To control flow operations, the electrical circuitry of the device **100** can include a flow control mechanism **170** with a comparator **170a,** a pump controller **170b,** and a pump driver **170c** arranged in a feedback control configuration. The comparator **170a** is configured to receive the differential pressure signal **175a** from the differential pressure sensor **150.** From this, the comparator **170a** is configured to subtract the differential pressure signal **175a** (i.e., the flow rate through the constrictor **140**) from a desired flow rate setpoint signal to produce or generate a flow rate error signal **175b** indicative of the difference between the desired flow rate and the actual flow rate. The pump controller **170b** is configured to amplify and process the flow rate error signal **175b** to produce or generate a pump control signal **175c,** which is used to driver a pump driver **170c.** The pump driver **170c** is configured to buffer the pump control signal **175c** to produce or generate a pump drive signal **175d,** which is transmitted to the pump **110** and used to drive the pump. If the differential pressure signal **175a** indicates that the flow rate is less than the desired flow rate, the resultant error signal **170b** indicates that the flow rate should be increased by increasing the speed of the pump **110.** When this occurs, the pump driver **170c** is configured to increase the speed of the pump **110.** Conversely, if the differential pressure signal **175a** indicates that the flow rate is greater than the desired flow rate, the resultant error signal **170b** indicates that the flow rate should be decreased by decreasing the speed of the pump **110.** When this occurs, the pump driver **170c** is configured to decrease the speed of the pump **110.** The pump controller **170b** is configured to control the pump **110** in a manner that will produce a stable, steady flow rate. Other types of feedback control of the pump **110** are contemplated. It is further contemplated to operate the pump **110** without feedback control, i.e. in open loop fashion.

As disclosed herein, a closed-loop control ripple cancellation device **180** is provided to cancel and thereby reduce or eliminate the pressure ripple introduced by the cyclical operation of the pump **110.** To provide pressure pulsation cancellation, the device **100** includes a pressure transducer **180c** (or other suitable device) which introduces a pressure ripple that is "opposite" that produced by the pump **110,** so as to cancel the pressure ripple of the pump **110.** The electronic circuitry **180a, 180b** is operatively connected to read the pressure sensor **150** and to drive the pressure transducer **180c** to inject ripple-canceling pressure pulses into flow of respired air to reduce or eliminate a pressure ripple in the flow of respired air. The ripple-canceling pressure pulses are determined by the electrical circuitry **180a, 180b** from the air pressure signal measured by the pressure sensor **150.** In other contemplated embodiments, gauge pressure measured by the gauge pressure sensor 160 is used, and those ripples are controlled instead. This may have an advantage in placing the ripple control driver closer to the capnography sensor. Cancellation of the pressure pulsations is accomplished with a closed-loop control ripple cancellation device **180** configured to attenuate or eliminate at least one pressure ripple in the respired air flowing through the constrictor **140.** The illustrative ripple cancellation device **180** includes a high-pass filter **180a,** a controller **180b** and the audio transducer (or similar air moving device) **180c.** An AC component of the differential pressure signal **175a** measured by the differential pressure sensor **150** is representative of the pressure pulsations created by the pump **110.** The high-pass filter **180a** is configured to receive the pressure value from the pressure sensor **150** and separate out and isolate this AC component of the signal to generate such that a pulsation or ripple signal **185a** that represents only those pulsations, without regard to a flow rate related component of the differential pressure signal **175a.** The cut-off frequency of the high-pass filter **180a** is chosen to pass the AC component corresponding to the pressure ripple. It will be appreciated that the high-pass filter **180a** may be replaced by a bandpass filter whose lower and upper cut-off frequencies are chosen such that the ripple signal is within the passband. On the other hand, the (lower) cut-off frequency of the bandpass filter **180a** should be high enough to remove the DC pressure component, so that the output of the filter **180a** corresponds to the pressure ripple component alone. The controller **180b** is programmed or tuned to produce or generate a transducer drive signal **185b** from the ripple signal to drive the audio transducer **180c.** The audio transducer **180c** produces or generates an antiphase pressure waveform **185c** from the transducer drive signal **185b** that counteracts and substantially nullifies the pressure pulsations (ripple) produced by the pump **110,** the goal of which being to produce minimal signal output from the high pass filter **180a.** The transducer **180c** is configured to apply the antiphase pressure waveform to air flowing from the constrictor **140** to the pump **110** to nullify the pulsations in the air. In some embodiments, the controller **180b** is a proportional-integral-derivative (PID) controller having proportional (P), integral (I), and derivative (D) parameters. The PID controller may be implemented using analog circuitry (e.g. op amps) and/or digital circuitry, e.g. a microprocessor or microcontroller. The controller **180b** can also be some other type of feedback controller (e.g. a PI controller).

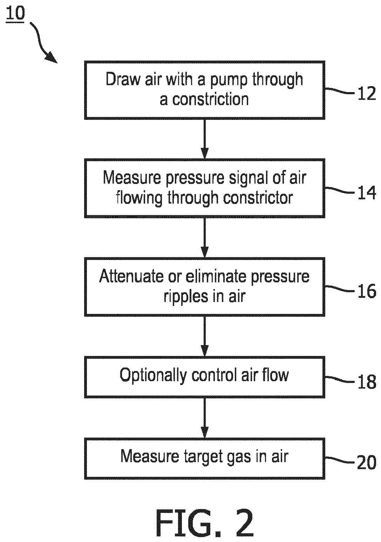

With reference now to FIGURE 2, the RGM device **100** is configured to perform a respiratory gas monitoring method **10.** At **12,** respired air is drawn, with the pump **110,** through the measurement area **130.** At least a portion of the respired air moves through the constrictor **140.** At **14,** a pressure signal **175a** of air flowing through the constrictor **140** is measured with the at least one pressure sensor **150.**

At **16,** at least one pressure ripple in the respired air flowing through the constrictor **140** is attenuated or eliminated with the ripple cancellation device **180a, 180b, 180c.** The attenuation or elimination includes separating, with the filter **180a,** an AC component of the pressure signal to generate a ripple signal **185a.** A transducer drive signal **185b** is generated from the ripple signal **185a** with the controller **180b.** An antiphase pressure waveform **185c** is generated from the transducer drive signal **185b** with the pressure transducer **180c.** The waveform **185c** is then applied by the transducers **180c** to air flowing from the constrictor **140** to the pump **110** to nullify the pulsations in the air. It should be noted that as the antiphase pressure waveform **185c** cancels the pressure ripple produced by the pump **110** within the sample tube segment **120c,** this cancellation also removes the pressure ripple produced by the pump **110** for all points "upstream" of the sample tube segment **120c,** particularly in the constrictor **140** and the further-"upstream" measurement area **130.**

At **18,** a flow of the air is optionally controlled with the flow control mechanism **170a, 170b, 170c.** (Note that operations **16** and **18** are performed concurrently). To do so, the comparator **170a,** is configured to subtract the pressure signal from a desired flow rate setpoint signal **175a** to generate a flow rate error signal **175b.** The pump controller **170b** is configured to amplify and process the flow rate error signal **175b** to produce or generate a pump control signal **175c,** which is used to drive a pump driver **170c.** The pump driver **170c** is configured to buffer the pump control signal **175c** to produce or generate a pump drive signal **175d,** which is transmitted to the pump **110** and used to drive the pump. When the differential pressure signal **175a** indicates that the flow rate is less than the desired flow rate, the pump driver **170c** is configured to increase the speed of the pump **110.** When the differential pressure signal **175a** indicates that the flow rate is greater than the desired flow rate, the pump driver **170c** is configured to decrease the speed of the pump **110.**

At **20,** a target gas in the flow of expired air is measured with the gauge pressure sensor **160.** (Again, operation **20** is performed concurrently with operations **16, 18**).

Referring back to FIGURE 1, the electrical circuitry of the device **100** (e.g., the flow control mechanism with a comparator **170a,** the pump controller **170b,** and the pump driver **170c,** the high-pass filter **180a,** and the controller **180b** and the electronic processor **196**) can be implemented as one or more microprocessors, microcontrollers, FPGA, or other digital device(s), and/or by analog circuitry.

It will be appreciated that the illustrative computational, data processing or data interfacing components of the device **100** may be embodied as a non-transitory storage medium storing instructions executable by an electronic processor (e.g., the electronic processor **196**) to perform the disclosed operations. The non-transitory storage medium may, for example, comprise a hard disk drive, RAID, or other magnetic storage medium; a solid state drive, flash drive, electronically erasable read-only memory (EEROM) or other electronic memory; an optical disk or other optical storage; various combinations thereof, or so forth.

The claims define the matter for which protection is sought in terms of the technical features of the invention.

## Claims

1. A respiration gas monitor device (**100**), comprising:
a pump (**110**) connected to draw a flow of respired air;
a pressure sensor (**150, 160**) connected to measure an air pressure signal responsive to the flow of respired air;
a pressure transducer (**180c**);
electrical circuitry (**170, 180**) operatively connected to measure flow across the pressure sensor, wherein the electrical circuity (**170, 180**) is operatively connected to read the pressure sensor and to drive the pressure transducer to inject ripple-canceling pressure pulses into flow of respired air to reduce or eliminate a pressure ripple in the flow of respired air wherein the ripple-canceling pressure pulses are determined by the electrical circuitry from the air pressure signal measured by the pressure sensor;
and
a gas component sensor (**190, 192, 194**) arranged to monitor a target gas in the flow of respired air.

2. The respiration gas monitor device according to claim 1,
wherein the electrical circuitry (**170, 180**) determines the ripple-canceling pressure pulses by operations including high-pass or bandpass filtering the air pressure signal measured by the pressure sensor (**150**).

3. The respiration gas monitor device according to either one of claims 1 and 2, further comprising a constrictor (**140**) comprising a capillary tube or an orifice;
wherein the pump (**110**) is connected to draw the flow of respired air through the constrictor and the pressure sensor (**150**) is connected to measure the air pressure signal indicating a pressure change across the constrictor.

4. The respiration gas monitor device according to any one of claims 1-3, wherein the electrical circuitry (**170, 180**) includes one of a proportional-integral-derivative (PID) controller and a microprocessor.

5. The respiration gas monitor according to any one of claims 1-4, wherein the gas component sensor includes:
an infrared light source (**190**) arranged to transmit infrared light through the flow of respired air;
a bandpass filter (**194**) arranged to filter the infrared light to pass a wavelength absorbed by the target gas, and
a light detector (**192**) arranged to detect the infrared light after being transmitted through the flow of respired air and filtered by the bandpass filter.

6. The respiration gas monitor according to any one of claims 1-5, wherein the pressure sensor (150, 160) is one of
(i) a differential pressure sensor (150) connected to measure differential pressure across a constrictor (140) in a path of the flow of respired air; or
(ii) a gauge pressure sensor (160) connected to measure a gauge pressure of the flow of respired air.

7. A device (**100**) for attenuating or eliminating pressure ripple in a respiration gas monitor, the device comprising:
a pump (**110**) configured to draw respired air from a measurement area (**130**);
a constrictor (**140**) through which at least a portion of the respired air drawn by the pump moves;
at least one pressure sensor (**150, 160**) configured to measure a pressure value of air flowing through the constrictor and to measure a differential pressure signal of air (**175a**) flowing through the constrictor (**140**), the at least one pressure sensor including a differential pressure sensor disposed at each of an inlet and an outlet of the constrictor; and
a ripple cancellation device (**180**) configured to attenuate or eliminate at least one pressure ripple in the respired air flowing through the constrictor, the ripple cancellation device further including
a filter (**180a**) configured to receive the pressure value from the pressure sensor and to separate an AC component of the pressure signal to generate a ripple signal **(185a),**
a controller (**180b**) configured to generate a transducer drive signal (**185b**) from the ripple signal, and
a pressure transducer (**180c**) configured to produce an antiphase pressure waveform (**185c**) from the transducer drive signal, and to apply the antiphase pressure waveform to air flowing from the constrictor (**140**) to the pump (**110**) to nullify the pulsations in the air.

8. The device (**100**) according to claim 7, further including a flow control mechanism (**170**) configured to control flow of air from the pump (**110**), the flow control mechanism including:
a comparator (**170a**) configured to receive the differential pressure signal (**175b**) from the differential pressure sensor (**150**), and subtract the differential pressure signal from a desired flow rate setpoint signal to generate a flow rate error signal (**175b**);
a pump controller (**170b**) configured to amplify and process the flow rate error signal to generate a pump control signal (**175c**); and
a pump driver (**170c**) configured to buffer the pump control signal to generate a pump drive signal (**175d**), and transmit the pump drive signal to the pump (**110**).

9. The device (**100**) according to claim 8, wherein
the pump driver (**170c**) is configured to increase the speed of the pump (**110**) when the differential pressure signal (**175a**) is less than the desired flow rate setpoint signal, and.
the pump driver (**170c**) is configured to decrease the speed of the pump (**110**) when the differential pressure signal (**175a**) is greater than the desired flow rate setpoint signal.

10. The device (**100**) according to any one of claims 7-9, wherein the pump (**110**) is configured to draw air from a patient first through a measurement area (**130**) and then through the constrictor (**140**), whereby the constrictor is disposed between the pump and measurement area.

11. A respiratory gas monitoring method (**10**), comprising:
drawing, with a pump (**110**), respired air through a measurement area (**130**), at least a portion of the respired air moving through a constrictor (**140**);
measuring, with at least one pressure sensor (**150, 160**), a pressure signal (**175a**) of air flowing through the constrictor;
attenuating or eliminating, with a ripple cancellation device (**180**), at least one pressure ripple in the respired air flowing through the constrictor, including separating, with a filter (**180a**), an AC component of the pressure signal to generate a ripple signal (**185a**), generating, with a controller (**180b**), a transducer drive signal (**185b**) from the ripple signal, and producing, with a pressure transducer (**180c**), an antiphase pressure waveform (**185c**) from the transducer drive signal, and apply the antiphase pressure waveform to air flowing from the constrictor (**140**) to the pump (**10**) to nullify the pulsations in the air; and
measuring, with a measurement device (**190, 192, 194**), a target gas in the flow of expired air.

12. The method (**10**) according to claim 11, wherein the measuring comprises: launching infrared light through the measurement area (**130**) using an infrared light source (**190**);
filtering the launched infrared light using a bandpass filter (**194**) having a passband encompassing an absorption line of the target gas,; and
detecting the launched and filtered infrared light using a light detector (**192**).

13. The method (**10**) according to any one of claims 11-12, further including:
subtracting, with a comparator (**170a**) the pressure signal (**175a**) from a desired flow rate setpoint signal to generate a flow rate error signal (**175b**);
amplifying and processing, with a pump controller (**170b**), the flow rate error signal to generate a pump control signal (**175c**); and
buffering, with a pump driver (**170c**), the pump control signal to generate a pump drive signal (**170d**), and transmit the pump drive signal to the pump (**110**).

14. The method (**10**) according to claim 13, further including:
with the pump driver (**170c**), increasing the speed of the pump (**110**) when the pressure signal (**175a**) is less than the desired flow rate setpoint signal; and
with the pump driver (**170c**), decreasing the speed of the pump (**110**) when the differential pressure signal (**175a**) is greater than the desired flow rate setpoint signal.

## Patentansprüche

1. Atemgasüberwachungsvorrichtung (100), umfassend:
eine Pumpe (110), die zum Ansaugen eines Atemluftstroms angeschlossen ist;
einen Drucksensor (150, 160), der angeschlossen ist, um ein Luftdrucksignal als Reaktion auf den Atemluftstrom zu messen;
ein Druckwandler (180c);
elektrische Schaltkreise (170, 180), die operativ verbunden sind, um den Durchfluss über den Drucksensor zu messen, wobei die elektrischen Schaltkreise (170, 180) operativ verbunden sind, um den Drucksensor auszulesen und den Druckwandler anzutreiben, um wellenunterdrückende Druckimpulse in den Atemluftstrom einzuspeisen, um eine Druckwelligkeit im Atemluftstrom zu reduzieren oder zu beseitigen, wobei die wellenunterdrückenden Druckimpulse von der elektrischen Schaltung aus dem vom Drucksensor gemessenen Luftdrucksignal bestimmt werden;
und
einen Gaskomponentensensor (190, 192, 194), der so angeordnet ist, dass er ein Zielgas im Atemluftstrom überwacht.

2. Atemgasüberwachungsvorrichtung nach Anspruch 1,
wobei der elektrische Schaltkreis (170, 180) die wellenunterdrückenden Druckimpulse durch Vorgänge bestimmt, die Hochpass- oder Bandpassfilterung des vom Drucksensor (150) gemessenen Luftdrucksignals umfassen.

3. Atemgasüberwachungsvorrichtung nach einem der Ansprüche 1 und 2, weiterhin umfassend eine Verengung (140), der ein Kapillarrohr oder eine Öffnung umfasst;
wobei die Pumpe (110) so angeschlossen ist, dass sie den Atemluftstrom durch die Verengung ansaugt, und der Drucksensor (150) so angeschlossen ist, dass er das Luftdrucksignal misst, das eine Druckänderung über der Verengung anzeigt.

4. Atemgasüberwachungsvorrichtung nach einem der Ansprüche 1 bis 3, wobei die elektrischen Schaltkreise (170, 180) einen Proportional-Integral-Differential (PID)-Regler oder einen Mikroprozessor umfassen.

5. Atemgasüberwachungsvorrichtung nach einem der Ansprüche 1 bis 4, wobei der Gaskomponentensensor umfasst:
eine Infrarotlichtquelle (190), die so angeordnet ist, dass sie Infrarotlicht durch den Atemluftstrom überträgt;
einen Bandpassfilter (194), der so angeordnet ist, dass er das Infrarotlicht so filtert, dass es eine vom Zielgas absorbierte Wellenlänge durchlässt, und
einen Lichtdetektor (192), der so angeordnet ist, dass er das Infrarotlicht erfasst, nachdem es durch den Atemluftstrom übertragen und durch den Bandpassfilter gefiltert wurde.

6. Atemgasüberwachungsvorrichtung nach einem der Ansprüche 1 bis 5, wobei der Drucksensor (150, 160) einer der folgenden ist:
(i) einen Differenzdrucksensor (150), der angeschlossen ist, um den Differenzdruck über eine Verengung (140) in einem Weg des Atemluftstroms zu messen; oder
(ii) einen Messdrucksensor (160), der angeschlossen ist, um einen Messdruck des Atemluftstroms zu messen.

7. Vorrichtung (100) zum Dämpfen oder Eliminieren von Druckwelligkeiten in einer Atemgasüberwachungsvorrichtung, wobei die Vorrichtung umfasst:
eine Pumpe (110), die so konfiguriert ist, dass sie Atemluft aus einem Messbereich (130) ansaugt;
eine Verengung (140), durch die sich mindestens ein Teil der von der Pumpe angesaugten Atemluft bewegt;
mindestens einen Drucksensor (150,160), der so konfiguriert ist, dass er einen Druckwert der durch die Verengung strömenden Luft und ein Differenzdrucksignal der durch die Verengung (140) strömenden Luft (175a) misst, wobei der mindestens eine Drucksensor einen Differenzdrucksensor umfasst, der jeweils an einem Einlass und einem Auslass der Verengung angeordnet ist; und
eine Welligkeitsunterdrückungsvorrichtung (180), die so konfiguriert ist, dass sie mindestens eine Druckwelligkeit in der Atemluft, die durch die Verengung strömt, abschwächt oder beseitigt, wobei die Welligkeitsunterdrückungsvorrichtung weiterhin Folgendes umfasst:
einen Filter (180a), der so konfiguriert ist, dass er den Druckwert vom Drucksensor empfängt und eine Wechselstromkomponente des Drucksignals abtrennt, um ein Welligkeitssignal (185a) zu erzeugen,
eine Steuerung (180b), die so konfiguriert ist, dass sie aus dem Welligkeitssignal ein Wandlerantriebssignal (185b) erzeugt, und
einen Druckwandler (180c), der so konfiguriert ist, dass er aus dem Wandlerantriebssignal eine gegenphasige Druckwellenform (185c) erzeugt und die gegenphasige Druckwellenform auf die Luft anwendet, die von der Verengung (140) zur Pumpe (110) strömt, um die Pulsationen in der Luft aufzuheben.

8. Vorrichtung (100) nach Anspruch 7, die außerdem einen Durchflusskontrollmechanismus (170) umfasst, der so konfiguriert ist, dass er den Luftstrom von der Pumpe (110) steuert, wobei der Durchflusskontrollmechanismus umfasst:
einen Komparator (170a), der so konfiguriert ist, dass er das Differenzdrucksignal (175b) vom Differenzdrucksensor (150) empfängt und das Differenzdrucksignal von einem gewünschten Durchflusssollwertsignal subtrahiert, um ein Durchflussfehlersignal (175b) zu erzeugen;
eine Pumpensteuerung (170b), die so konfiguriert ist, dass sie das Durchflussfehlersignal verstärkt und verarbeitet, um ein Pumpensteuersignal (175c) zu erzeugen; und
einen Pumpentreiber (170c), der so konfiguriert ist, dass er das Pumpensteuersignal puffert, um ein Pumpenantriebssignal (175d) zu erzeugen und das Pumpenantriebssignal an die Pumpe (110) zu übertragen.

9. Vorrichtung (100) nach Anspruch 8, wobei
der Pumpentreiber (170c) so konfiguriert ist, dass er die Drehzahl der Pumpe (110) erhöht, wenn das Differenzdrucksignal (175a) kleiner als das gewünschte Durchflusssollwertsignal ist, und
der Pumpentreiber (170c) so konfiguriert ist, dass er die Drehzahl der Pumpe (110) verringert, wenn das Differenzdrucksignal (175a) größer als das gewünschte Durchflusssollwertsignal ist.

10. Vorrichtung (100) nach einem der Ansprüche 7 bis 9, wobei die Pumpe (110) so konfiguriert ist, dass sie Luft von einem Patienten zuerst durch einen Messbereich (130) und dann durch die Verengung (140) ansaugt, wobei die Verengung zwischen der Pumpe und dem Messbereich angeordnet ist.

11. Atemgasüberwachungsverfahren (10), umfassend:
Ansaugen der Atemluft mit einer Pumpe (110) durch einen Messbereich (130), wobei sich mindestens ein Teil der Atemluft durch eine Verengung (140) bewegt;
Messen, mit mindestens einem Drucksensor (150, 160), eines Drucksignals (175a) der durch die Verengung strömenden Luft;
Abschwächen oder Beseitigen mindestens einer Druckwelligkeit in der Atemluft, die durch die Verengung strömt, mit einer Welligkeitsunterdrückungsvorrichtung (180), einschließlich Trennen einer Wechselstromkomponente des Drucksignals mit einem Filter (180a), um ein Welligkeitssignal (185a) zu erzeugen, Erzeugen eines Wandlerantriebssignals (185b) mit einer Steuerung (180b) aus dem Welligkeitssignal und Erzeugen, mit einem Druckwandler (180c), einer gegenphasigen Druckwellenform (185c) aus dem Wandlerantriebssignal, und Anwenden der gegenphasigen Druckwellenform auf Luft, die von der Verengung (140) zur Pumpe (10) strömt, um die Pulsationen in der Luft aufzuheben; und
Messen eines Zielgases im ausgeatmeten Luftstrom mit einem Messgerät (190, 192, 194).

12. Verfahren (10) nach Anspruch 11, wobei die Messung umfasst:
Einstrahlen von Infrarotlicht durch den Messbereich (130) unter Verwendung einer Infrarotlichtquelle (190);
Filtern des eingestrahlten Infrarotlichts unter Verwendung eines Bandpassfilters (194), dessen Durchlassband eine Absorptionslinie des Zielgases umfasst; und
Erfassen des eingestrahlten und gefilterten Infrarotlichts unter Verwendung eines Lichtdetektors (192) .

13. Verfahren (10) nach einem der Ansprüche 11 bis 12, weiterhin umfassend:
Subtrahieren des Drucksignals (175a) mit einem Komparator (170a) von einem gewünschten Durchflussmengensollwertsignal, um ein Durchflussmengenfehlersignal (175b) zu erzeugen;
Verstärken und Verarbeiten des Durchflussfehlersignals mit einer Pumpensteuerung (170b), um ein Pumpensteuersignal (175c) zu erzeugen; und
Puffern des Pumpensteuersignals mit einem Pumpentreiber (170c), um ein Pumpenantriebssignal (170d) zu erzeugen und das Pumpenantriebssignal an die Pumpe (110) zu übertragen.

14. Verfahren (10) nach Anspruch 13, weiter umfassend:
mit dem Pumpentreiber (170c) Erhöhen der Drehzahl der Pumpe (110), wenn das Drucksignal (175a) kleiner als das gewünschte Durchflussmengensollwertsignal ist; und
mit dem Pumpentreiber (170c), Verringern der Drehzahl der Pumpe (110), wenn das Differenzdrucksignal (175a) größer als das gewünschte Durchflussmengensollwertsignal ist.

## Revendications

1. Dispositif de surveillance des gaz respiratoires (100), comprenant:
une pompe (110) connectée pour aspirer un flux d'air respiré;
un capteur de pression (150, 160) connecté pour mesurer un signal de pression d'air en réponse au flux d'air respiré;
un transducteur de pression (180c);
un circuit électrique (170, 180) connecté de manière fonctionnelle pour mesurer le débit à travers le capteur de pression, où le circuit électrique (170, 180) est connecté de manière fonctionnelle pour lire le capteur de pression et pour entraîner le transducteur de pression afin d'injecter des impulsions de pression d'annulation d'ondulation dans le flux d'air respiré pour réduire ou éliminer une ondulation de pression dans le flux d'air respiré, où les impulsions de pression d'annulation d'ondulation sont déterminées par le circuit électrique à partir du signal de pression d'air mesuré par le capteur de pression;
et
un capteur de composant gazeux (190, 192, 194) agencé pour surveiller un gaz cible dans le flux d'air respiré.

2. Dispositif de surveillance des gaz respiratoires selon la revendication 1,
dans lequel le circuit électrique (170, 180) détermine les impulsions de pression d'annulation d'ondulation par des opérations comprenant un filtrage passe-haut ou passe-bande du signal de pression d'air mesuré par le capteur de pression (150).

3. Dispositif de surveillance des gaz respiratoires selon l'une ou l'autre des revendications 1 et 2, comprenant en outre un constricteur (140) comprenant un tube capillaire ou un orifice;
dans lequel la pompe (110) est connectée pour aspirer le flux d'air respiré à travers le constricteur et le capteur de pression (150) est connecté pour mesurer le signal de pression d'air indiquant un changement de pression à travers le constricteur.

4. Dispositif de surveillance des gaz respiratoires selon l'une quelconque des revendications 1 à 3, dans lequel le circuit électrique (170, 180) comprend l'un entre un dispositif de commande proportionnel-intégral-dérivé (PID) et un microprocesseur.

5. Dispositif de surveillance des gaz respiratoires selon l'une quelconque des revendications 1 à 4, dans lequel le capteur de composant gazeux comprend:
une source de lumière infrarouge (190) agencée pour transmettre une lumière infrarouge à travers le flux d'air respiré;
un filtre passe-bande (194) agencé pour filtrer la lumière infrarouge afin de laisser passer une longueur d'onde absorbée par le gaz cible, et
un détecteur de lumière (192) agencé pour détecter la lumière infrarouge après avoir été transmise à travers le flux d'air respiré et filtrée par le filtre passe-bande.

6. Dispositif de surveillance des gaz respiratoires selon l'une quelconque des revendications 1 à 5, dans lequel le capteur de pression (150, 160) est l'un entre :
(i) un capteur de pression différentielle (150) connecté pour mesurer la pression différentielle à travers un constricteur (140) dans un trajet du flux d'air respiré; ou
(ii) un capteur de pression relative (160) connecté pour mesurer une pression relative du flux d'air respiré.

7. Dispositif (100) pour atténuer ou éliminer l'ondulation de pression dans un dispositif de surveillance des gaz respiratoires, le dispositif comprenant:
une pompe (110) configurée pour aspirer l'air respiré d'une zone de mesure (130);
un constricteur (140) à travers lequel se déplace au moins une partie de l'air respiré aspiré par la pompe ;
au moins un capteur de pression (150, 160) configuré pour mesurer une valeur de pression de l'air s'écoulant à travers le constricteur et pour mesurer un signal de pression différentielle de l'air (175a) s'écoulant à travers le constricteur (140), l'au moins un capteur de pression comprenant un capteur de pression différentielle disposé au niveau d'une entrée et d'une sortie du constricteur; et
un dispositif d'annulation d'ondulation (180) configuré pour atténuer ou éliminer au moins une ondulation de pression dans l'air respiré s'écoulant à travers le constricteur, le dispositif d'annulation d'ondulation comprenant en outre
un filtre (180a) configuré pour recevoir la valeur de la pression du capteur de pression et pour séparer une composante CA du signal de pression pour générer un signal d'ondulation (185a),
un dispositif de commande (180b) configuré pour générer un signal de commande de transducteur (185b) à partir du signal d'ondulation, et
un transducteur de pression (180c) configuré pour produire une forme d'onde de pression antiphase (185c) à partir du signal de commande du transducteur, et pour appliquer la forme d'onde de pression antiphase à l'air s'écoulant du constricteur (140) vers la pompe (110) pour annuler les pulsations dans le air.

8. Dispositif (100) selon la revendication 7, comprenant en outre un mécanisme de commande de flux (170) configuré pour commander le flux d'air provenant de la pompe (110), le mécanisme de commande de flux comprenant:
un comparateur (170a) configuré pour recevoir le signal de pression différentielle (175b) provenant du capteur de pression différentielle (150), et soustraire le signal de pression différentielle d'un signal de consigne de débit souhaité pour générer un signal d'erreur de débit (175b);
un dispositif de commande de pompe (170b) configuré pour amplifier et traiter le signal d'erreur de débit afin de générer un signal de commande de pompe (175c); et
un pilote informatique de pompe (170c) configuré pour la mise en mémoire tampon du signal de commande de la pompe afin de générer un signal de commande de pompe (175d), et transmettre le signal de commande de pompe à la pompe (110).

9. Dispositif (100) selon la revendication 8, dans lequel
le pilote informatique de la pompe (170c) est configuré pour augmenter la vitesse de la pompe (110) lorsque le signal de pression différentielle (175a) est inférieur au signal de consigne de débit souhaité, et
le pilote informatique de pompe (170c) est configuré pour diminuer la vitesse de la pompe (110) lorsque le signal de pression différentielle (175a) est supérieur au signal de consigne de débit souhaité.

10. Dispositif (100) selon l'une quelconque des revendications 7 à 9, dans lequel la pompe (110) est configurée pour aspirer de l'air d'un patient d'abord à travers une zone de mesure (130) et puis à travers le constricteur (140), grâce à quoi le constricteur est disposé entre la pompe et la zone de mesure.

11. Procédé de surveillance des gaz respiratoires (10), comprenant:
l'aspiration, avec une pompe (110), de l'air respiré à travers une zone de mesure (130), au moins une partie de l'air respiré se déplaçant à travers un constricteur (140);
la mesure, avec au moins un capteur de pression (150, 160), d'un signal de pression (175a) de l'air s'écoulant à travers le constricteur;
l'atténuation ou l'élimination, avec un dispositif d'annulation d'ondulation (180), d'au moins une ondulation de pression dans l'air respiré s'écoulant à travers le constricteur, y compris la séparation, avec un filtre (180a), d'une composante CA du signal de pression pour générer un signal d'ondulation (185a), la génération, avec un dispositif de commande (180b), d'un signal de commande de transducteur (185b) à partir du signal d'ondulation, et la production, avec un transducteur de pression (180c), d'une forme d'onde de pression antiphase (185c) à partir du signal de commande du transducteur, et l'application de la forme d'onde de pression antiphase à l'air s'écoulant du constricteur (140) vers la pompe (10) pour annuler les pulsations dans l'air; et
la mesure, avec un dispositif de mesure (190, 192, 194), d'un gaz cible dans le flux d'air expiré.

12. Procédé (10) selon la revendication 11, dans lequel la mesure consiste à:
projeter une lumière infrarouge à travers la zone de mesure (130) en utilisant une source de lumière infrarouge (190);
filtrer la lumière infrarouge projetée en utilisant un filtre passe-bande (194) ayant une bande passante englobant une ligne d'absorption du gaz cible; et
détecter la lumière infrarouge projetée et filtrée en utilisant un détecteur de lumière (192).

13. Procédé (10) selon l'une quelconque des revendications 11 à 12, consistant en outre à:
soustraire, avec un comparateur (170a), le signal de pression (175a) d'un signal de consigne de débit souhaité pour générer un signal d'erreur de débit (175b);
amplifier et traiter, avec un dispositif de commande de pompe (170b), le signal d'erreur de débit pour générer un signal de commande de pompe (175c); et
mettre en mémoire tampon, avec un pilote informatique de pompe (170c), le signal de commande de la pompe pour générer un signal de commande de pompe (170d), et transmettre le signal de commande de la pompe à la pompe (110).

14. Procédé (10) selon la revendication 13, consistant en outre à:
avec le pilote informatique de la pompe (170c), augmenter la vitesse de la pompe (110) lorsque le signal de pression (175a) est inférieur au signal de consigne de débit souhaité; et
avec le pilote informatique de pompe (170c), diminuer la vitesse de la pompe (110) lorsque le signal de pression différentielle (175a) est supérieur au signal de consigne de débit souhaité.
